# EUROPÄISCHE PATENTSCHRIFT

(11) **EP 2 489 413 B1**
(45) Veröffentlichungstag und Bekanntmachung des Hinweises auf die Patenterteilung: **14.05.2014**
(21) Anmeldenummer: 12153504.1
(22) Anmeldetag: 01.02.2012
(51) Int. Cl.: A63B 21/008, A63B 23/18, A61M 16/08

(54) **Therapiegerät**
Therapy device
Appareil de thérapie

(30) Priorität: 21.02.2011 DE 102011011874
(43) Veröffentlichungstag der Anmeldung: 22.08.2012
(73) Patentinhaber: R. Cegla GmbH & Co. KG, 56410 Montabaur (DE)
(72) Erfinder: Cegla, Prof. Dr. Ulrich, 56410 Montabaur (DE)
(74) Vertreter: Engelhardt & Engelhardt

(56) Entgegenhaltungen:
- EP-A1- 0 262 239
- EP-A1- 2 087 927
- WO-A1-84/01704
- WO-A1-90/09203
- US-A1- 2009 159 062

## Beschreibung

Die Erfindung bezieht sich auf Therapiegerät nach den Oberbegriffen der Patentansprüche 1 und 11.

Ein derartiges Therapiegerät ist der EP 2 087 927 A1 zu entnehmen. Dieses Therapiegerät dient zur Verbesserung der Atmung eines Menschen. Dabei wird beim Einatmen die Atemluft in einen Schlauch eingepresst, der im Inneren eines gekrümmt ausgestalteten Rohrstückes eingesogen ist. Die Krümmung des Rohrstückes dient dazu, den Schlauch zu knicken, so dass dieser die Krümmung des Rohrstückes nachbildet und somit in einstellbare und unterschiedliche Schwingungsverhältnisse überführt werden kann.

Aufgrund der variabel einstellbaren Länge und Drehung des Schlauches im Inneren des Rohrstückes ist es möglich, die Druckzustände, durch die der Schlauch in oszillierende Schwingungen versetzt ist, variabel einzustellen und demnach die Druckwiderstände des Schlauches an die Bedürfnisse des jeweiligen Benutzers anzupassen. Folglich können mittels dieser Ausgestaltung des Therapiegerätes vom Asthmapatienten mit erheblichen Atemwegsproblemen bis zum Spitzensportler das Lungenvolumen erhöht und der Ausatmungsvorgang verbessert werden.

Zwar haben sich solche Atmungs-Therapiegeräte in der Praxis bewährt, jedoch hat sich herausgestellt, dass es einen erheblichen medizinisch bedingten Bedarf gibt, solche Atmungs-Therapiegeräte nicht nur für das Einatmen sondern auch für das Einatmen bereitzustellen.

Der Erfindung liegt daher die Aufgabe zugrunde, ein Atmungs-Therapiegerät zu schaffen, das gleichzeitig beim Ein- und beim Ausatmen verwendet werden kann, so dass der Patient mit Hilfe des erfindungsgemäßen Atmungs-Therapiegerätes die Atemwege sowohl beim Aus- als auch beim Einatmen trainieren kann und dadurch die Atmung verbessert ist.

Darüber hinaus ist es Aufgabe der Erfindung, ein Atmungs-Therapiegerät bereitzustellen, mittels dem ein Benutzer wahlweise bzw. kontinuierlich Ein- und Ausatmen kann, um die entsprechenden Therapieerfolge zu erzielen, ohne dass das Atmungs-Therapiegerät abzusetzen ist.

Diese Aufgaben werden durch die Merkmale des kennzeichnenden Teils von Patentanspruch 1 gelöst.

Weitere vorteilhafte Weiterbildungen der Erfindung ergeben sich aus den Unteransprüchen.

Dadurch, dass in dem Mundstück eine Kanalabzweigung vorgesehen ist, ist an diese ein zweites Rohrstück anschließbar, in dem eine den Luftaustritt behindernde oder freigebende Durchgangsöffnung angeordnet ist, so dass mittels der einstellbaren Strömungsverhältnisse vorteilhafterweise eine Anpassung des Luftwiderstandes an die Bedürfnisse und Therapieerfolge des Benutzers erfolgt. Folglich kann ein derartiges Kombi-Atmungs-Therapiegerät sowohl zum Ein- als auch zum Ausatmen verwendet werden.

Wenn an dem zweiten Rohrstück ein weiterer Schlauch eingesetzt ist, wirkt dieser beim Einatmen als Ventil, denn die entstehenden Druckzustände im Inneren des zweiten Rohrstückes führen dazu, dass der Schlauch aufgrund des Unterdruckes zusammengezogen ist, so dass keine Luft durch diesen hindurch tritt. Der Schlauch wirkt demnach nach Art eines Verschlussventils.

Beim Ausatmen dagegen wird der Schlauch in dem zweiten Rohrstück aufgeweitet, so dass die Atemluft durch diesen hindurchströmt und den Schlauch in oszillierende Schwingungen versetzt. Gleichzeitig ist der in dem ersten Rohrstück eingesetzte Schlauch aufgrund des in dem Inneren des Rohrstückes herrschenden Überdruckes zusammengepresst und wirkt nach Art eines Ventils als Stöpsel oder Luftverschlusselement. Gleichwohl wird aufgrund des in dem ersten Rohrstück vorherrschenden Überdruckes der in dieses eingesetzte Schlauch in Schwingungen versetzt, so dass der Benutzer aufgrund des sich einstellenden Druckzustandes und der damit in Wirkverbindung stehenden Schwingverhaltens des ersten Schlauches hörbar feststellen kann, welche Atmungserfolge sich beim Einatmen ergeben.

Ein erfindungsgemäßes Ausführungsbeispiel ist in der Zeichnung dargestellt, das nachfolgend näher erläutert ist. Im Einzelnen zeigt:
- Figur 1: ein Kombi-Atmungs-Therapiegerät, bestehend aus zwei parallel zueinander gekrümmt ausgestalteten Rohrstücken, in denen jeweils versetzt zueinander ein im Inneren des jeweiligen Rohrstückes schwingender Schlauch angeordnet ist und mit einem die beiden Rohrstücke verbindenden Mundstück, in dem eine Kanalabzweigung eingearbeitet ist, in perspektivischer Ansicht,
- Figur 2a: das Atmungs-Therapiegerät gemäß Figur 1, im Zustand des Einatmens und mit den sich dadurch ergebenden Strömungsverhältnisse,
- Figur 2b: das Atmungs-Therapiegerät gemäß Figur 1, im Zustand des Ausatmens und mit den sich daraus ergebenden Strömungsverhältnisse,
- Figur 3a: ein in das erste Rohrstück eingesetzte Ringelement gemäß Figur 1, mit in dieses eingearbeiteten Durchgangsöffnungen und mit einer Verstellbuchse zur Abdeckung der Durchgangsöffnungen, in Explosionsdarstellung,
- Figur 3b: das Ringelement gemäß Figur 3a, im Längs- und Querschnitt und
- Figur 3c: ein Luftverteilelement, das in dem zweiten Rohrstück gemäß Figur 1 eingesetzt ist, im Längsschnitt.

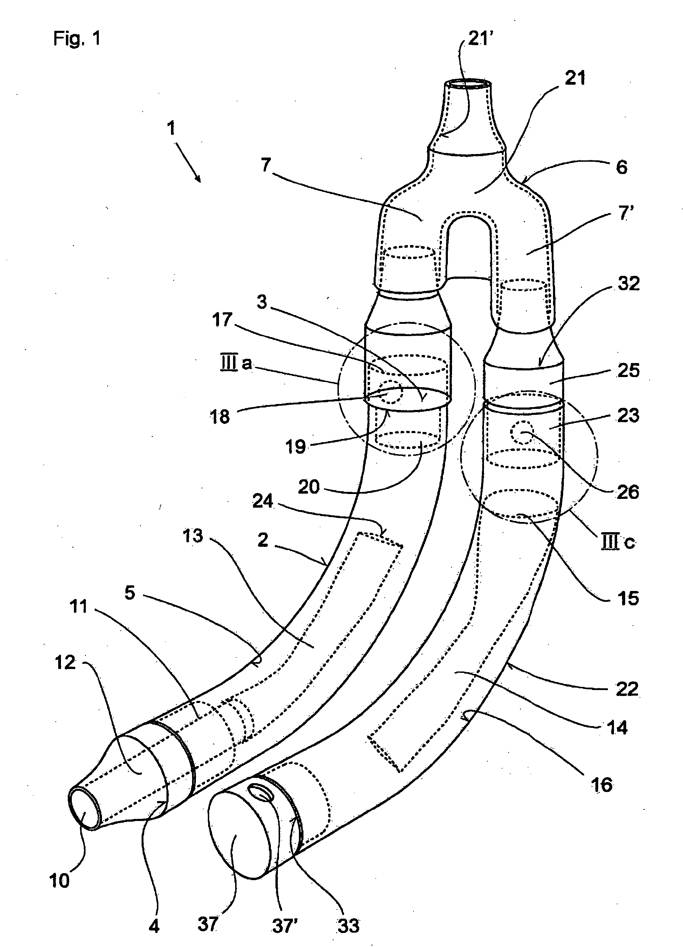

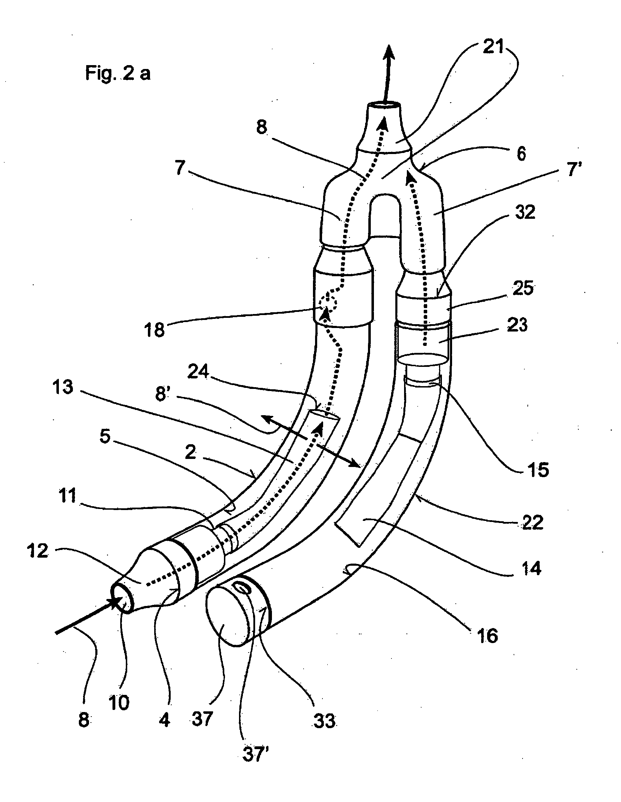

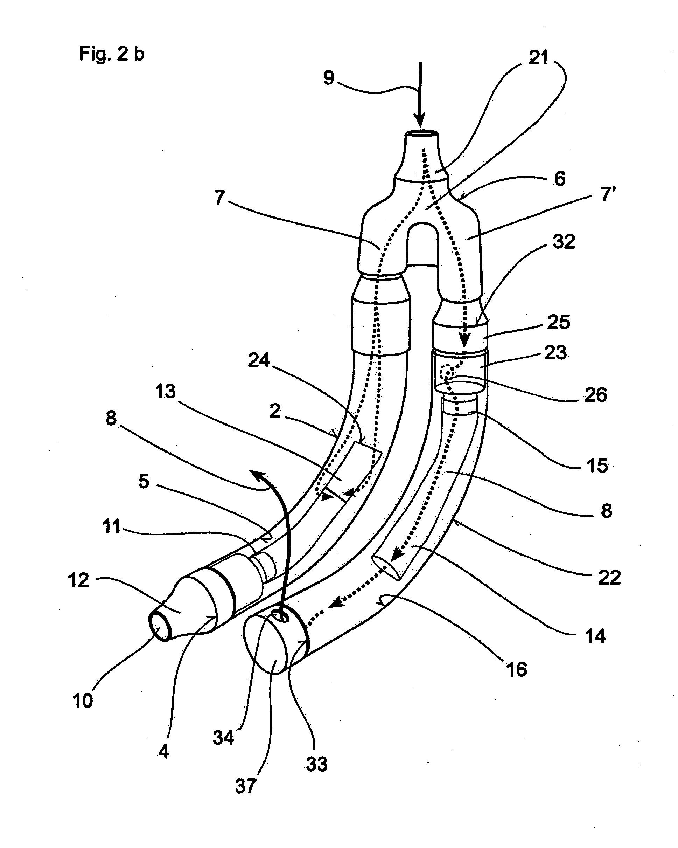

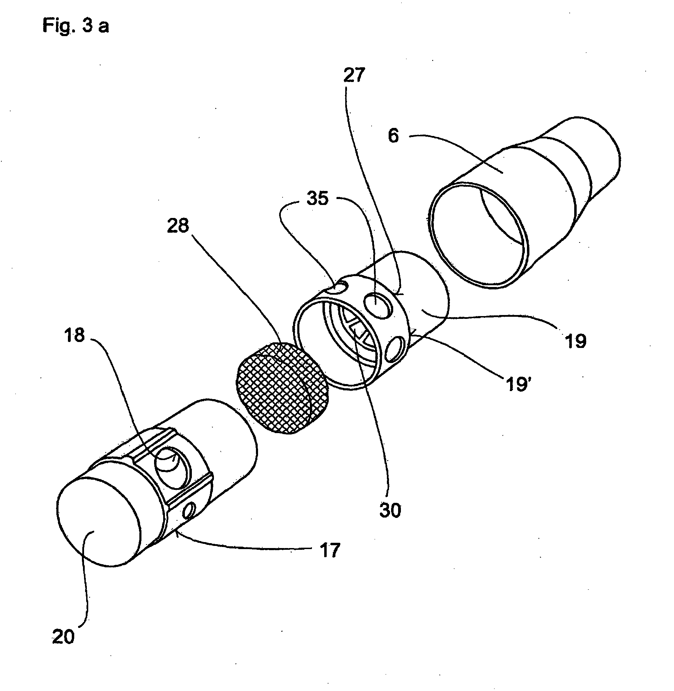

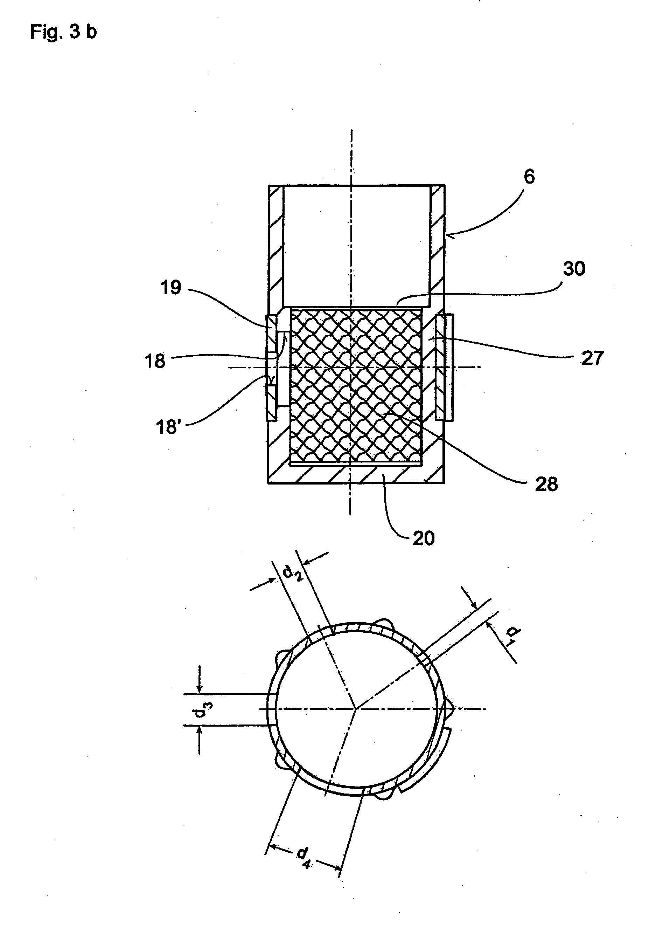

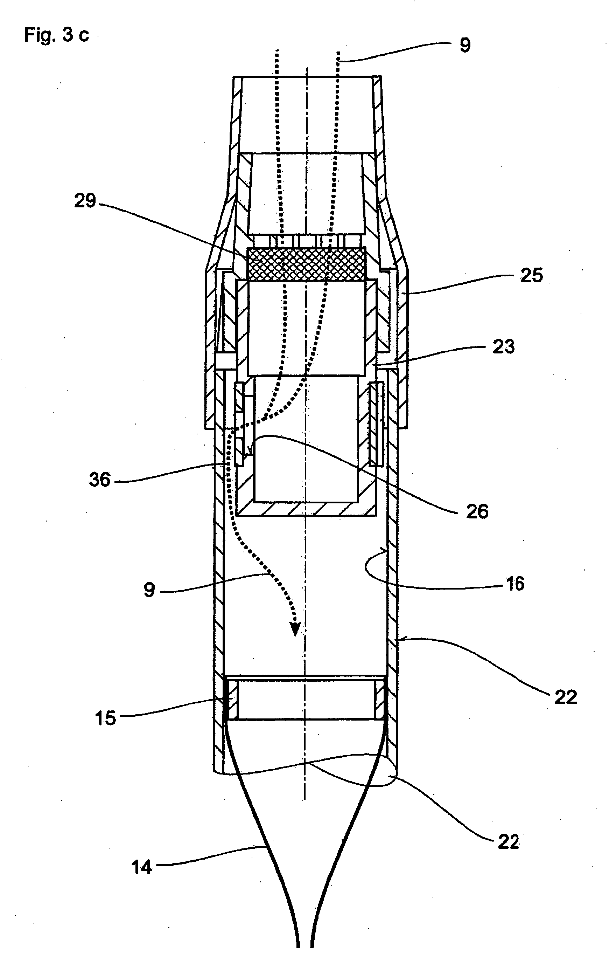

In Figur 1 ist ein Therapiegerät 1 zur Verbesserung der Atmung eines Patienten abgebildet, das gemäß den Figuren 2a und 2b zum gleichzeitigen Ein- und Ausatmen und damit zu einer kombinierten Atemwegstherapie verwendbar ist. Zunächst soll anhand der Figuren 1, 3a, 3b und 3c der konstruktive Aufbau des Therapiegerätes 1 und anschließend durch die Figuren 2a und 2b die Funktionsweise des Therapiegerätes 1 erläutert sein.

Das Therapiegerät 1 besteht zunächst aus einem ersten Rohrstück 2, das gekrümmt oder abgeknickt ausgestaltet ist. Parallel zu dem ersten Rohrstück 2 ist ein zweites Rohrstück 22 vorgesehen.

In dem ersten Rohrstück 2 ist in dessen ersten Stirnseite 3 ein Mundstück 6 ein- oder aufgesetzt, durch das die beiden Rohrstücke 2 und 22 miteinander verbunden sind. In dem Mundstück 6 ist nämlich eine Kanalabzweigung 21 vorgesehen, durch die zwei Luftkanäle 7 bzw. 7' des Mundstückes 6, die mit den beiden Rohrstücken 2 und 22 kommunizieren, zu einem Ausgangs- bzw. Eingangskanal 21' zusammengefüge sind.

In dem ersten Rohrstück 2 ist in dessen zweiten Stirnseite 4, die von dem Mundstück 6 abgewandt ist, eine Einlassöffnung 10 vorgesehen, in die ein Haltezapfen 11 derart befestigbar ist, dass der Haltezapfen 11 in unterschiedlichen Positionen bezogen auf die Stirnseite 4 des Rohrstückes 2 positionierbar ist. An dem Haltezapfen 11 ist nämlich ein erster Schlauch 13 angebracht, der fest mit dem Haltezapfen 11 verbunden ist. In den Haltezapfen 11 ist ein Durchlasskanal 12 eingearbeitet, der mit dem Schlauch 13 derart in kommunizierender Wirkverbindung steht, dass die durch die Einlassöffnung 10 einströmende Luft durch den Durchlasskanal 12 in den ersten Schlauch 13 gelangt und diesen aufweitet und bei ausreichender Luftströmung in oszillierende Schwingungen versetzt, so dass der im Inneren des ersten Rohrstückes 2 positionierte Schlauch 13, und zwar insbesondere dessen freies dem Mundstück 6 zugewandtes Ende 24, zwischen der Innenwand 5 des Rohrstückes 2 hin und her bewegt ist.

Den Figuren 1 und 3a kann des weiteren entnommen werden, dass in das erste Rohrstück 2, zwischen dem freien Ende 24 des Schlauches 13 und dem Mundstück 6, ein Ringelement 17 eingesetzt ist, das zum Einen vier in Umfangsrichtung eingearbeitete Durchgangsöffnungen 18 oder Schlitze und zum Anderen eine senkrecht zu der Symmetrieachse des Rohrstückes 2 ausgerichtete Prallplatte 20 aufweist, durch die die Luftströmung im inneren des Rohrstückes 2 in Richtung der Innenwand 5 des Rohrstückes 2 und damit in Richtung der Durchgangsöffnungen 18 umgelenkt ist.

Darüber hinaus ist eine Verstellbuchse 19 vorgesehen, die im Inneren ein Gitter 30 aufweist, auf das ein Filter 28 aufgesetzt ist. Folglich wird der Filter 28 durch die Verstellbuchse 19 gehalten. Die Verstellbuchse 19 ist dem Mundstück 6 zugewandt und in dieses reibschlüssig gehalten und abgestützt. Das Gitter 30 kann auch im Mundstück 6 zum Halten des Filters 28 vorgesehen sein.

Durch die Verstellbuchse 19 können die Durchgangsöffnungen 18 ganz oder teilweise verschlossen oder vollständig freigegeben sein, denn an die Verstellbuche 19 ist ein umlaufender Bund 19' angeformt, in den eine oder mehrere Öffnungen 35 eingearbeitet sind, deren Durchmesser unterschiedlich groß ausgestaltet sind und deren Innenkontur verschiedenartig zueinander ausgebildet sein kann. Die Durchgangsöffnungen 18 des Ringelementes 17 können durch den Bund 19' verschlossen sein, diese teilweise freigeben bzw. eine vollständige Freigabe der Durchgangsöffnungen 18 ist einstellbar, wenn also die Öffnungen 35 des Bundes 19' der Verstellbuchse 19 fluchtend zu einer oder mehreren der Durchgangsöffnungen 18 des Ringelementes 17 ausgerichtet sind.

Der Filter 28 dient dazu, die durch diesen hindurchströmende Luft zu reinigen und Schwebstoffe bzw. Mikropartikel aufzufangen und zu konservieren.

Des Weiteren besteht das Therapiegerät 1 aus einem in dem zweiten Rohrstück 22 angeordneten Luft-Verteilelement 23, das im Bereich des Mundstückes 6 positioniert ist. Das Luft-Verteilelement 23 ist insbesondere in der Figur 3c abgebildet und ist ringförmig ausgestaltet, so dass dieses mit der Innenkontur des Mundstückes 6 und dem Rohrstück 22 korrespondiert und in diese eingesteckt werden kann.

In dem Luft-Verteilelement 23 sind Durchgangsöffnungen eingearbeitet, deren Durchmesser unterschiedlich groß ausgestaltet sind, und zwar wie in Figur 3b im

Querschnitt gezeigt. Von der kleinsten Größe D1 erstrecken sich die geometrischen Verhältnisse bis zu dem größten Durchmesser D4. Es ist möglich, eine Vielzahl von unterschiedlich groß bemessenen Durchgangsöffnungen 26 in das Luft-Verteilelement 23 einzuarbeiten.

Auch das Luft-Verteilelement 23 weist eine senkrecht zu der Symmetrieachse des zweiten Rohrstückes 22 ausgerichtete Prallplatte 20 auf. Auf der gegenüberliegenden Seite der Prallplatte 20 ist an dem Luft-Verteilelement das Gitter 30 vorgesehen, auf dem ein zweiter Filter 29 gehalten ist. Das Gitter 30 und der Filter 29 sind für die Ausatmung optional einbaubar.

Gemäß Figur 3c ist in dem zweiten Rohrstück 22 in dieses mittels eines Halterings 15 ein zweiter Schlauch 14 eingesetzt. Der Haltering 15 ist beabstandet zu dem Luft-Verteilelement 23 angeordnet, so dass zwischen diesen beiden Bauteilen ein ausreichend groß bemessener Zwischenraum im Inneren des Rohrstückes 22 gegeben ist.

Dem Luft-Verteilelement 23 ist ein Stellring 27 zugeordnet, der verdrehbar an diesem gehalten ist und in den eine Vielzahl von Öffnungen 35 mit unterschiedlich groß bemessenen Durchmessern bzw. Öffnungsweiten eingearbeitet ist. Durch Verdrehen des Stellringes 27 können somit die Öffnungen 35 des Stellringes 27 mit dem in dem Luft-Verteilelement 23 vorgesehenen Durchgangsöffnungen 18 in unterschiedlichen Strömungsquerschnitten eingestellt sein, so dass die Luft teilweise beim Durchströmen behindert ist. Die einströmende Luft soll nämlich durch diese aus dem Luft-Verteilelement 23 in einen Zwischenraum 36, der zwischen der Innenwand 16 des Rohrstückes 22 und dem Luft-Verteilelement 23 bzw. dem Außenumfang Stellringes 27 hindurchströmen.

Aus Figur 1 ist zudem ersichtlich, dass das zweite Rohrstück 22 eine zweite Stirnseite 33 aufweist, die mittels eines Stöpsels 37 verschlossen sein kann, in den eine Öffnung 37' eingearbeitet ist.

In Figur 2a ist zunächst der Einatmungsvorgang schematisch dargestellt. Die sich einstellende Strömungsrichtung der eingesaugten Luft ist mit der Bezugsziffer 8 gekennzeichnet. Der Patient atmet folglich mittels seiner Atemmuskulatur Luft durch das erste Rohrstück 2 ein, das in dieses, und zwar im Bereich der zweiten Stirnseite 4, durch die Einlassöffnung 10 einströmt und durch den Durchlasskanal 12 des Haltezapfens 11 in den ersten Schlauch 13 eintritt.

Das freie Ende 24 des Schlauches 13 ist gemäß den schematischen Schwingungspfeilen 8' zwischen der Innenwand 5 des Rohrstückes 2 hin und her bewegt. Aufgrund der unterschiedlichen Krümmung des Rohrstückes 2 und der relativ zu dieser einstellbaren Position des Schlauches 13 kann das Schwingungsverhalten des Schlauches 13 variabel eingestellt werden. Da der Haltezapfen 11 nämlich verschiebbar in der zweiten Stirnseite 4 des Rohrstückes 2 gehalten ist, kann die Länge des Schlauches 13, die in das Rohrstück 2 eintaucht, unterschiedlich lang bemessen sein, so dass der Schlauch 13 an unterschiedlichen Stellen ausgeknickt bzw. umgebogen sein kann.

Durch die einströmende bzw. eingesaugte Luft 8 weitet sich der biegsame Schlauch 13 auf, so dass die Luft durch diesen hindurch tritt und in das Innere des Rohrstückes 2 gelangt. An der Prallplatte 20 des Ringelementes 17 wird die Luft nach außen, also in Richtung der Innenwand 5, umgelenkt und von dieser seitlich geführt in Richtung auf die Durchgangsöffnung 18 bzw. Öffnungen 35 des Ringelementes 17 bzw. der Verstellbuchse 19 geleitet.

Durch die eingestellte Querschnittsfläche zwischen der Durchgangsöffnung 18 und der Öffnung 35 erhöht sich oder reduziert sich der dort vorherrschende Luftwiderstand in Abhängigkeit von der Querschnittsfläche, die von der Luftströmung 8 durchströmt werden kann.

Sobald die Luftströmung 8 das Ringelement 17 in Richtung des Mundstückes 6 verlässt, strömt diese durch den ersten Durchlasskanal 7 in Richtung des gemeinsamen Kanals 21' der Kanalabzweigung 21 in den Mundraum des Patienten. Aufgrund der vorherrschenden Luftdrucksituation im Inneren der beiden Rohrstücke 2 und 22 entsteht im zweiten Rohrstück 22 ein Unterdruck, denn aus diesem wird die Luft gemäß der Luftströmungsrichtung 8 abgesaugt. Dieser Unterdruck wird über die zwischen dem Luft-Verteilelement 23 und dem Inneren des zweiten Rohrstückes 22 vorherrschende Luftdrucksituation an den zweiten Schlauch 14 weitergeleitet, wodurch im Inneren des zweiten Schlauches 14 ein Unterdruck entsteht, durch den die biegsame Seitenwand des Schlauches 14 aneinanderliegen und somit einen Luftdurchtritt durch den zweiten Schlauch 14 verhindern. Folglich wirkt der zweite Schlauch 14 in diesem Betriebszustand als eine Art Ventil, durch das der Lufteintritt durch das zweite Rohrstück 22 in Richtung des Mundstückes 6 verhindert ist.

Aufgrund des Schwingungsverhaltens des ersten Schlauches 13 kann der Patient hörbar feststellen, dass der mittels Öffnungsweite voreingestellte notwendige Unterdruck erreicht wurde..

In Figur 2b ist der Betriebszustand des Ausatmens gezeigt. Die Strömungsrichtung der ausgeatmeten Luft aus dem Lungenvolumen des Patienten ist mit der Bezugsziffer 9 gekennzeichnet. Die Luftströmung 9 wird zunächst in das Mundstück 6 eingepresst und dort im Bereich der Luftkanalabzweigung 21 verteilt. Die Luftströmung die in das zweite Rohrstück 22 eindringt, wird über die Durchgangsöffnungen 26 in den Zwischenraum 36 eingepresst und gelangt von diesem in das Innere des zweiten Rohrstückes 22 und damit in den zweiten Schlauch 14, der durch die Luftströmung 9 nunmehr aufgeweitet ist und seinerseits in ein oszillierendes Schwingverhalten überführt ist, wenn durch die eingepresste Luft ein ausreichend groß bemessener Luftdruck erzeugt wird.

Die durch den zweiten Schlauch 14 austretende Luft 9 wird über die zweite Stirnseite 33 und die Öffnung 37' des Stöpsels 37 ins Freie abgegeben.

Der andere ausgeatmete Teil der Luftströmung 9 gelangt in das erste Rohrstück 2 und wird dort zunächst das Ringelement 17 durch die Durchgangsöffnungen 18 verlassen und in das Innere des ersten Rohrstückes 2 eintreten. In diesem herrscht aufgrund der eingeströmten Luft ein Überdruck, durch den der erste Schlauch 13 zusammengepresst ist, wodurch dieser nach Art eines Ventils verschlossen ist, so dass aus dem Inneren des ersten Rohrstückes 2 keine Luft entweichen kann.

Der vorherrschende Überdruck im ersten Rohrstück 2 führt demnach dazu, dass unmittelbar nach dem Ausatmen ein entsprechend ausgebildetes Luftpolster vorherrscht, durch das die ausgeatmete Luft 9 nach einer bestimmten Zeit zumindest vollständig in das zweite Rohrstück 22 geleitet ist. Das erste und zweite Rohrstück 2 und 22 können mit separaten Mundstücken 6 versehen sein und demnach unabhängig voneinander eingesetzt werden.

## Patentansprüche

1. Therapiegerät (1) zur Verbesserung der Atmung eines Patienten, mit einem gekrümmt oder abgeknickt gestalteten Rohrstück (2), in dessen erste Stirnseite (3) ein mit einem Durchlasskanal (7) versehenes Mundstück (6) eingesetzt ist, mit einem in die zweite Stirnseite (4) des Rohrstückes (2) fest mit diesem verbundenen Haltezapfen (11), in den ein Durchlasskanal (12) eingearbeitet ist und der ganz oder teilweise in das Innere des Rohrstückes (2) einsteckbar ist, und mit einem an dem Haltezapfen (11) angebrachten biegsamen Schlauch (13), der im Inneren des Rohrstückes (2) angeordnet ist und dessen freies Ende (24) im Bereich des Mundstückes (6) zwischen einer Innenwand (5) des Rohrstückes (2) frei beweglich ist,
**dadurch gekennzeichnet,**
**dass** in den Durchlasskanal (7) des Mundstückes (6) eine Kanal-Abzweigung (21) einmündet, an die ein zweites Rohrstück (22) angeschlossen ist, und dass in das zweite Rohrstück (22) ein Luft-Verteilelement (23) eingesetzt ist, in dessen Außenumfang mindestens eine Durchgangsöffnung (26) eingearbeitet ist, deren Öffnungsweite mittels eines drehbar gelagerten Stellrings (27) variabel einstellbar ist.

2. Therapiegerät nach Anspruch 1,
**dadurch gekennzeichnet.**
**dass** in das,zweite Rohrstück (22) ein dem Luft-Verteilelement (23) in Strömungsrichtung (8) nachgeschalteter biegsamer Schlauch (14) eingesetzt ist, durch den ein durch das Luft-Verteilelement (23) strömender Luftstrom (8) geführt ist, durch den der Schlauch (14) in Schwingungen versetzt ist.

3. Therapiegerät nach Anspruch 2,
**dadurch gekennzeichnet**
**dass** der Schlauch (14) an dem Luft-Verteilelement (23) unmittelbar oder mittels eines Halteringes (15) an der Innenwand (16) des zweiten Rohrstückes (22) arretiert ist.

4. Therapiegerät nach einem der vorgenannten Ansprüche,
**dadurch gekennzeichnet,**
**dass** in dem Luft-Verteilelement (23) ein Filter (28) eingesetzt ist, durch den die Luftströmung (8) hindurchströmt und dass der Filter (28) an einem Gitter- oder Lochblech (30) des Luft-Verteilelementes (23) aufliegt und von diesem abgestützt ist.

5. Therapiegerät nach einem der vorgenannten Ansprüche,
**dadurch gekennzeichnet,**
**dass** dem Luft-Verteilelement (23) ein Abdeckring (31) zugeordnet ist, der in Umfangsrichtung des Luft-Verteilelements (23) drehbar ist und durch den das oder die Durchgangsöffnungen (26), die in das Luft-Verteilelement (23) eingearbeitet sind, ganz oder teilweise verschlossen oder vollständig freigegeben sind.

6. Therapiegerät nach Anspruch 1,
**dadurch gekennzeichnet,**
**dass** in das erste Rohrstück (2) im Bereich der dem Mundstück (6) zugewandten Stirnseite (3) ein Ringelement (17) eingesetzt ist, dass in das Ringelement (17) mindestens eine Durchgangsöffnung (18) eingearbeitet ist und dass dem Ringelement (17) eine Verstellbuchse (19) im montierten Zustand übergestülpt ist, durch die die jeweilige Durchgangsöffnung (18) ganz oder teilweise verschlossen oder vollständig freigegeben ist.

7. Therapiegerät nach Anspruch 6,
**dadurch gekennzeichnet,**
**dass** an dem Ringelement (17) eine Prallplatte (20) angeförmt ist, durch die die Luftströmung (9) in Richtung des Rohrstückes (2) blockiert ist.

8. Therapiegerät nach Anspruch 1,
**dadurch gekennzeichnet,**
**dass** der Abstand zwischen dem freien Ende (24) des Schlauches (13) und dem in das Rohrstück (2) eingesetzten Mundstückes (6) variabel einstellbar ist und/oder dass die Länge des Schlauches (13) durch ein Verschieben des Haltezapfens (11) relativ zu dem Rohrstück (2) zur Veränderung des Krümmungsradius des Schlauches (13) verschiebbar ist.

9. Therapiegerät nach einem der vorgenannten Ansprüche,
**dadurch gekennzeichnet,**
**dass** in dem inneren des zweiten Rohrstückes (22) beim Einatmen ein Unterdruck entsteht, durch den der zweite Schlauch (14) im inneren des zweiten Rohrstückes (22) in Schwingung versetzt ist und gleichzeitig nach Art eines Ventils verschlossen ist.

10. Therapiegerät nach einem der vorgenannten Ansprüche,
**dadurch gekennzeichnet,**
**dass** beim Ausatmen mittels eines in dem ersten Rohrstück (2) entstehenden Überdruckes der erste Schlauch (13) nach Art eines Ventils verschlossen ist.

## Claims

1. A therapy device (1) for improving the respiration of a patient, with a curved or kinked pipe section (2), into the first end (3) of which a mouthpiece (6) provided with a passage channel (7) is inserted, with a holding peg (11) inserted into the second end (4) of the pipe section (2) and firmly connected to it, the holding peg (11) having a passage channel (12) worked into it and being insertable in whole or in part into the inside of the pipe section (2), and with a flexible hose (13) attached to the holding peg (11) that is arranged inside the pipe section (2), with the free end (24) of the hose (13) able to move freely in the area of the mouthpiece (6) between an inner wall (5) of the pipe section (2),
**characterised in that**,
a channel branch (21) emerges into the passage channel (7) of the mouthpiece (6) and has a second pipe section (22) connected to it, and that the second pipe section (22) has an air distribution element (23) inserted into it, into the outside circumference of which at least one passage opening (26) is worked, the opening width of which can be variably adjusted by means of a setting ring (27).

2. The therapy device in accordance with Claim 1,
**characterised in that**,
a flexible hose (14) behind the air distribution element (23) in the flow direction (8) is inserted in the second pipe section (22), by means of which an air flow (8) through the air distribution element (23) is guided and as a result of which the hose (14) is induced to vibrate.

3. The therapy device in accordance with Claim 2,
**characterised in that**,
the hose (14) on the air distribution element (23) is locked directly or by means of a holding ring (15) against the inside wall (16) of the second pipe section (22).

4. The therapy device in accordance one of the aforementioned claims,
**characterised in that**,
the air distribution element (23) has a filter (28) inserted in it, through which the air flow (8) flows, and that the filter (28) lies on a mesh or perforated plate (30) of the air distribution element (23) and is supported by the same.

5. The therapy device in accordance with one of the aforementioned claims,
**characterised in that**,
the air distribution element (23) has a covering ring (31) allocated to it, which can be rotated in the peripheral direction of the air distribution element (23) and by means of which the passage opening(s) (26) worked into the air distribution element (23) are fully or partially closed or fully opened.

6. The therapy device in accordance with Claim 1,
**characterised in that**,
the first pipe section (2) has a ring element (17) inserted into it in the area of the end (3) facing towards the mouthpiece (6), that at least one passage opening (18) is worked into the ring element (17) and that an adjustable sleeve (19) is folded over the ring element (17) in the installed condition, by means of which the corresponding passage opening (18) is fully or partially closed or fully opened.

7. The therapy device in accordance with Claim 6,
**characterised in that**,
a baffle plate (20) is formed onto the ring element (17), by means of which the air flow (9) is blocked in the direction of the pipe section (2).

8. The therapy device in accordance with Claim 1,
**characterised in that**,
the distance between the free end (24) of the hose (13) and the mouthpiece (6) inserted in the pipe section (2) is variably adjustable and/or that the length of the hose (13) can be moved relative to the pipe section (2) by moving the holding peg (11) in order to change the curvature radius of the hose (13).

9. The therapy device in accordance with one of the aforementioned claims,
**characterised in that**,
a negative pressure is produced in the inside of the second pipe section (22) during inhalation, by means of which the second hose (14) inside the second pipe section (22) is induced to vibrate and, at the same time, is closed in the way of a valve.

10. The therapy device in accordance with one of the aforementioned claims,
**characterised in that**,
during exhalation, the first hose (13) is closed in the way of a valve by means of the positive pressure prevailing in the first pipe section (2).

## Revendications

1. Appareil de thérapie (1) servant à améliorer la respiration d'un patient, avec une pièce tubulaire (2) courbée ou plié, dans la première face frontale (3) de laquelle il est inséré une embouchure (6) munie d'un canal de passage (7), avec, dans la deuxième face frontale (4) de la pièce tubulaire (2), un téton de retient solidaire (11) dans lequel il est un canal de passage (12) et qui entre partiellement ou complètement dans l'intérieur de la pièce tubulaire (2), et avec un tuyau flexible (13) prévu sur le téton de retient (11) s'étendant à l'intérieur de la pièce tubulaire (2), dont l'extrémité libre (24) peut se déplacer librement au niveau de l'embouchure (6) entre la paroi intérieure (5) de la pièce tubulaire (2),
**caractérisé en ce que**
dans le canal de passage (7) de l'embouchure (6), il débouche une dérivation (21), sur laquelle il est branché une deuxième pièce tubulaire (22) et que dans la deuxième pièce tubulaire (22), il est inséré un élément de distribution d'air (23) dans le pourtour extérieur duquel il est pratiqué au moins une ouverture de passage (26) dont la taille de l'ouverture se laisse régler à l'aide d'un anneau de réglage (27) logé de manière pivotant.

2. Appareil de thérapie d'après la revendication 1,
**caractérisé en ce que**
dans la deuxième pièce tubulaire (22), il est inséré un tuyau flexible (14) qui en direction du flux (8), est disposé en aval de l'élément de distribution d'air (23) et qui dirige un flux d'air (8) traversant l'élément de distribution d'air (23), le flux d'air faisant osciller le tuyau flexible (14).

3. Appareil de thérapie d'après la revendication 2,
**caractérisé en ce que**
le tuyau flexible (14) est fixé directement sur l'élément de distribution d'air (23) ou, moyennant un anneau de retient (15), sur la paroi intérieure (16) de la deuxième pièce tubulaire (22).

4. Appareil de thérapie d'après une des revendications précédentes,
**caractérisé en ce que**
dans l'élément de distribution d'air (23), il est inséré un filtre (28) par lequel traverse le flux d'air (8) et que le filtre (28) porte sur une grille ou une tôle perforée (30) de l'élément de distribution d'air (23) et est appuyé par celui-ci.

5. Appareil de thérapie d'après une des revendications précédentes,
**caractérisé en ce que**,
à l'élément de distribution d'air (23), il est assigné un anneau de recouvrement (31) pivotant en direction du pourtour de l'élément de distribution d'air (23), et qui permet de recouvrir partiellement ou entièrement l'ouverture ou les ouvertures de passage (26) pratiquée(s) dans l'élément de distribution d'air (23).

6. Appareil de thérapie d'après la revendication 1,
**caractérisé en ce que**
dans la première pièce tubulaire (2), il est inséré un élément annulaire (17) au niveau de la face frontale (3) donnant sur l'embouchure (6), que dans l'élément annulaire (17), il est pratiqué au moins une ouverture de passage (18) et que, en état monté, il est retroussé sur l'élément annulaire (17) une douille de réglage (19) qui permet de recouvrir partiellement ou entièrement l'ouverture de passage respective (18).

7. Appareil de thérapie d'après la revendication 6,
**caractérisé en ce que**
sur l'élément annulaire (17), il est pratiqué une chicane (20) bloquant le flux d'air (9) en direction de la pièce tubulaire (2).

8. Appareil de thérapie d'après la revendication 1,
**caractérisé en ce que**
l'écartement entre l'extrémité libre (24) du tuyau flexible (13) et l'embouchure (6) insérée dans la pièce tubulaire (2) se laisse régler et/où que la longueur du tuyau flexible (13) se laisse régler par le déplacement du téton de retient (11) relativement par rapport à la pièce tubulaire (2) en vue du changement du rayon de courbure du tuyau flexible (13).

9. Appareil de thérapie d'après une des revendications précédentes,
**caractérisé en ce que**,
dans l'intérieur de la deuxième pièce tubulaire (22), il se forme, à la respiration, une dépression qui fait osciller le deuxième tuyau flexible (14) à l'intérieur de la deuxième pièce tubulaire (22) et qui le ferme simultanément à la façon d'une soupape.

10. Appareil de thérapie d'après une des revendications précédentes,
**caractérisé en ce que**
moyennant une surpression se formant au moment de l'expiration dans la première pièce tubulaire (2), le premier tuyau flexible (13) est fermé à la façon d'une soupape.
